# EUROPEAN PATENT SPECIFICATION

(11) **EP 0 783 507 B1**
(45) Date of publication and mention of the grant of the patent: **04.06.2003**
(21) Application number: 94925701.8
(22) Date of filing: 28.07.1994
(51) Int. Cl.: C07D 513/04, C07D 498/04, C07D 517/04, C07D 333/38, C07D 417/06, A61K 31/54

(54) **COMPOUNDS USEFUL AS ANTIPROLIFERATIVE AGENTS AND GARFT INHIBITORS**
VERBINDUNGEN DIE ALS ANTIPROLIFERIERENDE MITTEL UND GARFT INHIBITOREN VERWENDBAR SIND
COMPOSES UTILES COMME AGENTS ANTIPROLIFERATIFS ET INHIBITEURS DE LA GLYCINAMIDE RIBONUCLEOTIDE FORMYLE TRANSFERASE (GARFT)

(43) Date of publication of application: 16.07.1997
(73) Proprietor: AGOURON PHARMACEUTICALS, INC., La Jolla, CA 92037 (US)
(72) Inventor: VARNEY, Michael, D., Carlsbad, CA 92009 (US); ROMINES, William, H., San Diego, CA 92130 (US); PALMER, Cynthia, L., LaMesa, CA 91941 (US)
(74) Representative: Grünecker, Kinkeldey, Stockmair & Schwanhäusser Anwaltssozietät
(86) International application number: US9408522
(87) International publication number: WO96003407

(56) References cited:
- EP-A- 0 438 261
- WO-A-86/05181
- WO-A-94/13295
- US-A- 4 123 550
- JOURNAL OF HETEROCYCLIC CHEMISTRY., vol.20, no.6, 1983, PROVO US pages 1557 - 1560 J. L. CASARRUBIO ET AL 'On the syntheses of thiophene analogs of practorol and reversed practorol'

## Description

### Background of the Invention

The present invention relates to compounds as defined below that inhibit the enzyme glycinamide ribonucleotide formyl transferase (GARFT). The invention also relates to pharmaceutical compositions containing these compounds. The compounds have antitumor, antiinflammatory, antipsoriatic and/or immunosuppressive activity. The invention is also related to the preparation of these compounds described.

The large class of antiproliferative agents includes antimetabolite compounds. A particular subclass of antimetabolites known as antifolates or antifoles are antagonists of the vitamin folic acid. Typically, antifolates closely resemble the structure of folic acid and incorporate the characteristic P-benzoyl glutamate moiety of folic acid. The glutamate moiety of folic acid takes on a double negative charge at physiological pH. Therefore, this compound and its analogs have an active energy driven transport system to cross the cell membrane and exert a metabolic effect.

GARFT is a folate-dependent enzyme in the *de novo* purine biosynthesis pathway. This pathway is critical to cell division and proliferation. Shutting down this pathway is known to have an antiproliferative effect, in particular, an antitumor effect. Thus, a number of folate analogs have been synthesized and studied for their ability to inhibit GARFT. A prototypical specific tight-binding inhibitor of GARFT, 5,10-dideazatetrahydrofolic acid, has been reported to show antitumor activity. See F.M. Muggia, "Folate antimetabolites inhibitor to *de novo* purine synthesis," *New Drugs, Concepts and Results in Cancer Chemotherapy,* Kluwer Academic Publishers, Boston (1992), 65-87.

Recently, condensed heterocyclic glutamic acid derivatives useful as antiproliferative agents or GARFT inhibitors have been disclosed in International Application PCT/US94/00418, filed by this applicant on January 18, 1994. The present invention continues the progress in this art through the development of further useful antiproliferative agents and GARFT inhibitors.

### Summary of the Invention

The present invention relates to compounds as defined below. These compounds are effective in inhibiting glycinamide ribonucleotide formyl transferase (GARFT) and the growth and proliferation of cells of higher organisms and of microorganisms such as bacteria, yeast and fungi. The invention further relates to pharmaceutical compositions containing these compounds or suitable salts thereof and the use of these compounds as inhibitors of the enzyme GARFT.

As indicated above, compounds of the invention possess antiproliferative activity, a property which can express itself in the form of antitumor activity. A compound of the invention can be active *per se*, or as a precursor converted *in vivo* to an active compound. Preferred compounds of the invention are especially active in inhibiting the enzyme GARFT. Particularly preferred compounds are active in inhibiting the growth of the L1210 cell line, a mouse leukemia cell line that can be grown in tissue culture. Compounds of the invention can also be active in inhibiting the growth of bacteria such as *Escherichia coli* gram-negative bacteria which can be grown in culture.

The compounds according to the invention, as well as the pharmaceutically acceptable salts thereof, may be incorporated into convenient dosage forms, such as capsules, tablets and injectable preparations. Solid or liquid pharmaceutically acceptable carriers, diluents or excipients may also be employed.

Solid carriers include starch, lactose, calcium sulfate dihydrate, terra alba, sucrose, talc, gelatin, agar, pectin, acacia, magnesium stearate and stearic acid. Liquid carriers include syrup, peanut oil, olive oil, saline solution and water.

The carrier or diluent may include any prolonged-release material, such as glyceryl monostearate or glyceryl distearate, alone or with wax. When a liquid carrier is used, the preparation may be in the form of a syrup, elixir, emulsion, soft gelatin capsule, sterile injectable liquid (e.g. solution) or a nonaqueous or aqueous liquid suspension.

The pharmaceutical preparations are prepared following conventional techniques of the pharmaceutical chemist involving steps such as mixing, granulation and compressing when necessary for tablet forms, or mixing, filling and dissolving the ingredients as appropriate to give the desired products for oral, parenteral, topical, intravaginal, intranasal, intrabronchial, intraocular, intraaural or rectal administration.

The compositions of the invention may further comprise one or more other pharmaceutically active compounds. For example, one of the following antitumor agents may be included in the composition: mitotic inhibitors (e.g., vinblastine); alkylating agents; dihydrofolate reductase inhibitors or TS inhibitors; antimetabolites (for example, 5-fluorouracil, cytosinerabinoside); intercalating antibiotics (for example, adriamycin, bleomycin) ; enzymes (for example, asparaginase); topoisomerase inhibitors (for example, etoposide); and biological response modifiers (for example, interferon). The compositions of the invention may also comprise another GARFT inhibitor or antiproliferative agent, such as a compound described in commonly assigned international Publication No. WO 94/13295, published June 23, 1994, or International Publication No. WO 92/05153, published April 2, 1992, The compositions of the invention may also comprise one or more antibacterial, antifungal, antiparasitic, antiviral, antipsoriatic or anticoccidial agents. Exemplary antibacterial agents include: sulfonamides, such as sulfamethoxazole, sulfadiazine, sulfameter and sulfadoxine; dihydrofolic reductase inhibitors, such as trimethoprim, bromodiaprim and trimetrexate; penicillins; cephalosporins; and the quinolone carboxylic acids and their fused isothiazolo analogs.

Another aspect of the invention relates to a therapeutic method of inhibiting the growth or proliferation of cells of higher organisms or microorganisms, which comprises administering to a host an effective amount or quantity of a compound according to the present invention. The compounds of the invention are particularly useful in the treatment of mammalian hosts, such as human hosts, and in the treatment of avian hosts. A particularly preferred therapeutic process comprises administering to a host an amount of a compound according to the present invention effective to inhibit GARFT.

Many of the antiproliferative compounds described herein and their pharmaceutically acceptable salts thereof can be employed in the therapeutic process of the invention. The compounds may be administered in the form of a pharmaceutically acceptable composition comprising a diluent or carrier as described above.

A dose of a composition contains at least an effective quantity of the active compound and preferably is made up of one or more pharmaceutical dosage units. An "effective quantity" can be a quantity sufficient to inhibit the folate metabolic pathways and derive the beneficial effects therefrom, e.g., through administration of one or more of the pharmaceutical dosage units.

An exemplary daily dose for a vertebrate host comprises an amount of up to one gram active compound per kilogram of the host, preferably one-half of a gram, more preferably 100 milligrams, and most preferably, about 50 milligrams or less, per kilogram of the host's body weight. The selected dose may be administered to a warmblooded animal or mammal, for example, a human patient in need of treatment mediated by folate metabolic pathways inhibition, by any suitable method of administrating the dose including: topically, for example, as an ointment or cream; orally; rectally, for example, as a suppository; parenterally by injection; or continuously by intravaginal, intranasal, intrabronchial, intraaural or intraocular infusion.

The compounds according to the invention produce any one or more of an antiproliferative effect, an antibacterial effect, an antiparasitic effect, an antiviral effect, an antipsoriatic effect, an antiprotozoal effect, an anticoccidial effect, an antiinflammatory effect, an immunosuppressive effect and an antifungal effect. The compounds are especially useful in producing an antitumor effect in a vertebrate host harboring a tumor.

### Detailed Description of the Invention

In particular, the present invention relates to antiproliferative compounds of the Formula below 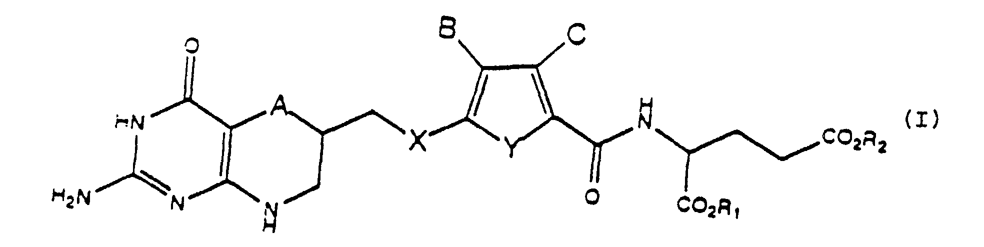 wherein:
A is S
X is CH₂
Y is S
   and
R₁ and R₂ are independently hydrogen. The invention further relates to pharmaceutically acceptable salts of the compounds of the present invention. The invention also relates to intermediates for making such compounds.

Although the compounds of the present invention are shown in the 4-oxo form and are referred to as such throughout this description, the oxo group exists in tautomeric equilibrium with the corresponding 4-hydroxy group. It will therefore be understood that the term "compounds of the present invention means the structurally depicted 4-oxo and the tautomeric 4-hydroxy forms. Thus, the invention also relates to pharmaceutically acceptable salts of the 4-hydroxy tautomers of the compounds depicted by the present invention.

One embodiment of the present invention relates to a compound of the formula

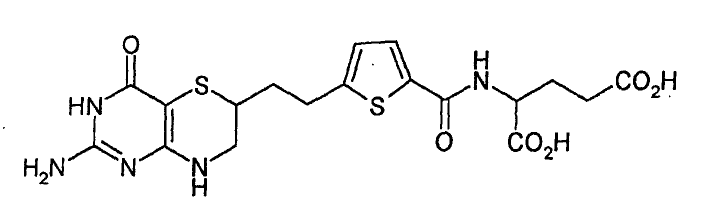

In a preferred embodiment the invention relates to a compound as defined above, which is optically active. In a more preferred embodiment, the compound as defined above has the the following structure:

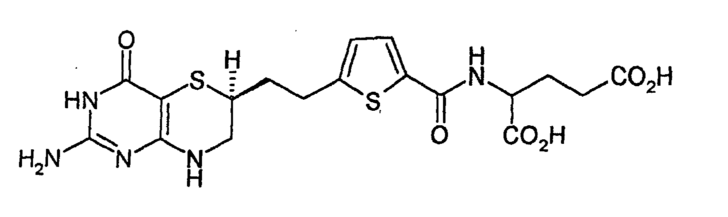

In another preferred embodiment, the compound as defined above has the the following structure:

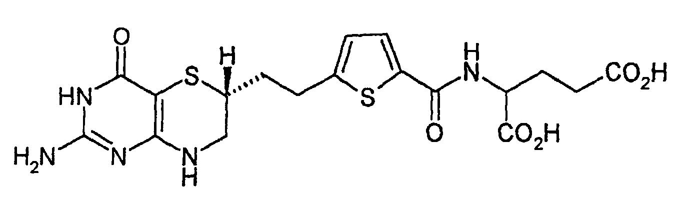

Preferably, X is CH₂, Y is preferably S. Preferably, R₁ and R₂ are independently selected from hydrogen, most preferably, from hydrogen. It is particularly preferred that A and Y are each sulfur and X is CH₂

The compounds of the present invention are useful as GARFT inhibitors. The compounds of the present invention in which R₁ and R₂ are each hydrogen are especially active antitumor or antiproliferative agents.

The pharmaceutically acceptable salts of the invention include, for example, alkaline metal, alkaline earth metal, other non-toxic metals, and ammonium and substituted ammonium salts of the glutamic acid compounds of the invention. Exemplary salts include sodium, potassium, lithium, calcium, magnesium, pyridinium and substituted pyridinium salts of the free acid compounds.

The compounds of the invention can be prepared as follows. A useful starting material for preparing compounds of the present invention wherein X is CH₂ is a compound of the Formula II: 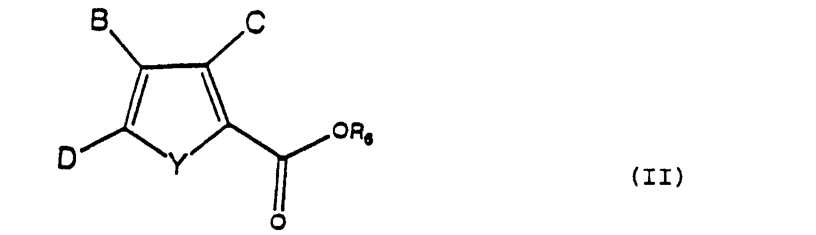 wherein B, C and Y are as defined above for the compoud of the present invention D is Cl, Br or I; and R₆ is a hydrogen or a moiety that forms with the attached CO₂ a readily hydrolyzable ester group. Preferably, D is bromo or iodo. Preferably, R₆ is hydrogen, C₁₋C₆ alkyl, hydroxyalkyl, alkylaryl or arylalkyl, more preferably, hydrogen or C₁-C₂ alkyl.

The compound of the Formula II is reacted with a compound of the Formula III: 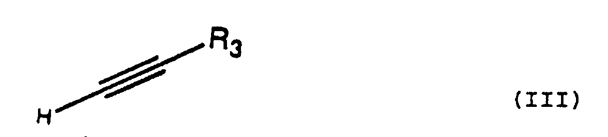 wherein R₃ is a substituted or unsubstituted C₁-C₆ alkyl group, or a trisubstituted silyl group. Preferably, R₃ is CH₂OH or trimethylsilyl.

The reaction of the compounds of the Formulae II and III is carried out in the presence of a suitable transition-metal catalyst, preferably palladium or nickel, a non-nucleophilic auxiliary base, preferably a substituted amine, in a solvent in which at least one of the reactants is at least partially soluble under conditions sufficient to obtain a compound of the Formula IV: 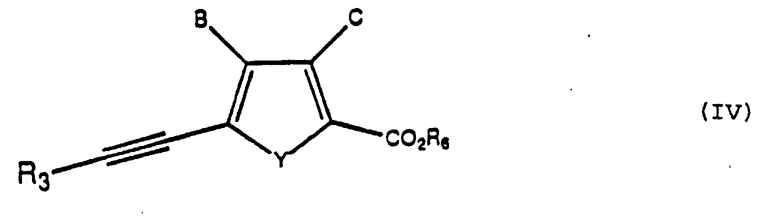 wherein B, C and Y are as defined above for compound of the present invention R₆ is as defined above for Formula II; and R₃ is as defined above for Formula III.

When R₃ is a trisubstituted silyl group, the silyl group is preferably removed with a nucleophilic base, such as methanolic or ethanolic potassium carbonate, or a fluoride salt, such as potassium fluoride, cesium fluoride or tetrabutylammonium fluoride, in a solvent in which at least one of the reactants is at least partially soluble (e.g., methanol, dimethylformamide, ethanol, dimethylacetamide, dimethylsulfoxide or isopropanol) to give a compound of the Formula V: 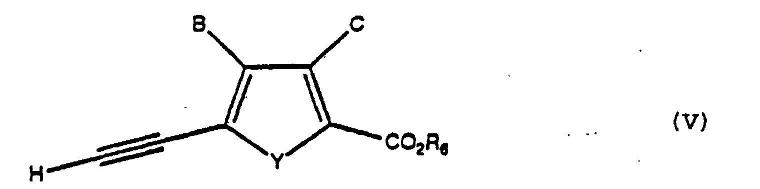 wherein B, C and Y are as defined above for compoud of the present invention and R₆ is as defined above for Formula II.

The compound of Formula V is reacted with an electrophile, preferably an N-protected glycinal, more preferably N-t-butoxycarbonylglycinal or bis-N-t-butoxycarbonylglycinal, under basic conditions using a non-nucleophilic base, preferably lithium bis-trimethylsilylamide, potassium bis-trimethyl-silylamide, sodium bis-trimethylsilylamide or lithium diisopropylamide, at a low temperature, preferably from -90°C to 25°C, in a suitable solvent in which one of the reactants is at least partially soluble, preferably tetrahydrofuran, diethyl ether or dioxane, to give a compound of the Formula VI: 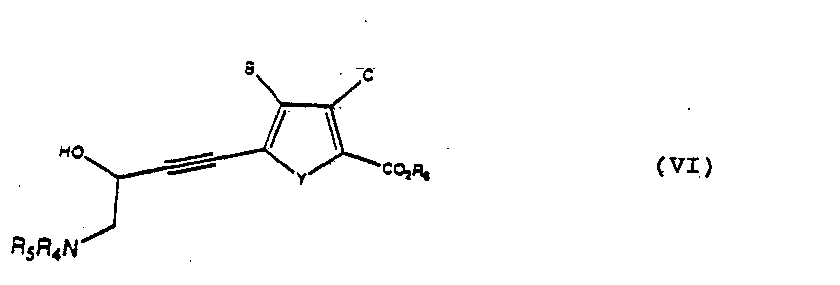 wherein B, C and Y are as defined above for the compound of the present invention R₆ is as defined above for Formula II; and R₄ and R₅ are each independently hydrogen or a readily removable nitrogen-protecting group. R₄ and R₅ are preferably hydrogen, t-butoxycarbonyl, benzyloxycarbonyl or benzyl.

The compound of the Formula VI is then reduced, preferably with hydrogen gas in the presence of a suitable metal catalyst, to obtain a compound of the Formula VII: 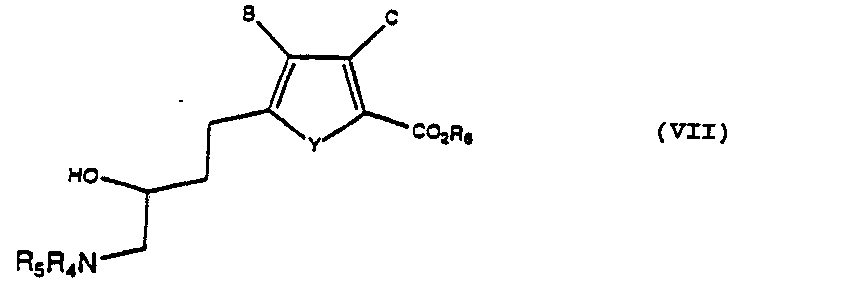 wherein B, C and Y are as defined above for the compound of the present invention R₆ is as defined above for Formula II; and R₄ and R₅ are as defined above for Formula VI.

The compound of the Formula VII is then reacted with an acylating or sulfonylating agent, preferably methanesulfonyl chloride or p-toluenesulfonyl chloride, in the presence of a non-nucleophilic base, preferably triethylamine or diisopropylethylamine, in a suitable solvent in which at least one of the reactants is at least partially soluble, to obtain an activated hydroxy group. The activated hydroxy group is displaced with a suitable nucleophile, preferably a thioacid salt, more preferably potassium thioacetate, to obtain a compound of the Formula VIII: 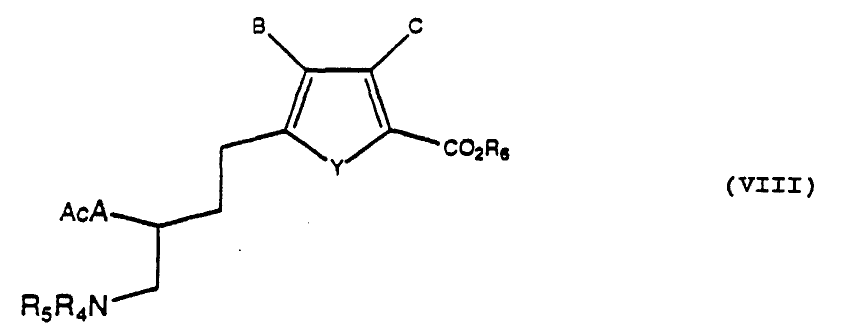 wherein A, B, C and Y are as defined above for the compound of the present invention R₆ is as defined above for Formula II; R₄ and R₅ are as defined above for Formula VI; and Ac is an acyl group, preferably acetyl.

Alternatively, the compound of Formula VII is converted to the compound of Formula VIII in one chemical operation using triphenylphosphine, diethyl or dimethyl azadicarboxylate, and an acidic nucleophile, preferably thioacetic acid, in a suitable solvent.

The compound of the Formula VIII is treated with a nucleophilic base, preferably potassium carbonate, sodium carbonate, sodium hydroxide or potassium hydroxide, in an alcoholic solvent, preferably methanol, ethanol or isopropanol, in the presence of an alkylating agent, preferably dimethyl or diethyl chloromalonate, to obtain a compound of the Formula IX: 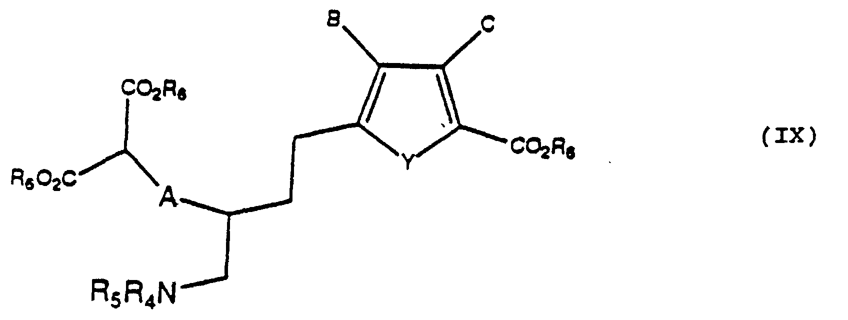 wherein A, B, C and Y are as defined above for the compoud of the present invention R₆ is as defined above for Formula II; and R₄ and R₅ are as defined above for Formula VI.

The compound of the Formula IX is treated under conditions suitable to remove either or both of the R₄ and R₅ protecting groups to produce a compound of the Formula X: 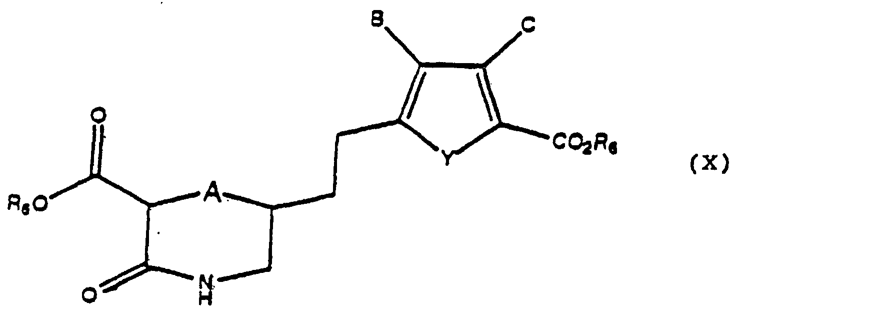 wherein A, B, C and Y are as defined above for the compound of the present invention and R₆ is as defined above for Formula II. Where t-butoxycarbonyl (t-BOC) is a protecting group, the conditions for removal of this group are preferably treatment with trifluoroacetic acid followed by neutralization to produce the compound of the Formula X.

The compound of the Formula X is reacted with an alkylating agent, preferably trimethyl or triethyl oxonium tetrafluoroborate, in a suitable solvent, preferably dichloromethane, to form an intermediate lactim ether. The intermediate lactim ether is reacted with guanidine in an alcoholic solvent, preferably methanol, ethanol or isopropanol, to form a compound of the Formula XI: 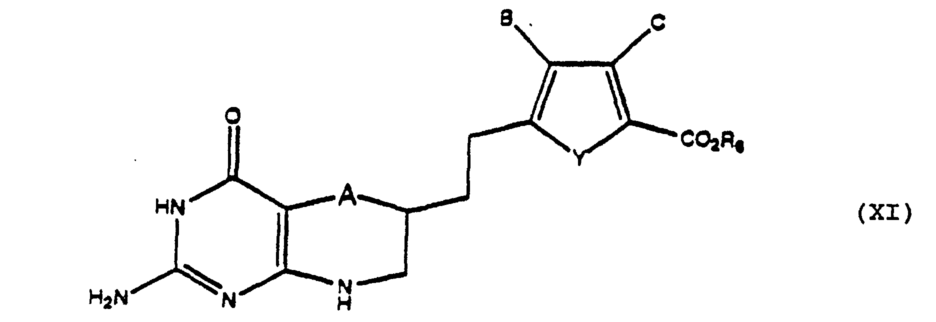 wherein A, B, C and Y are as defined above for the compound of the present invention and R₆ is as defined above for Formula II.

6 Alternatively, the compound of the Formula X is converted to the compound of the Formula XI by reacting the compound of the Formula X with a thiolating agent, preferably P₂S₅ or 2,4-bis(4-methoxyphenyl)-1,3-dithia-2,4-diphosphetane-2,4-disulfide, to form the thiolactam intermediate. This can then be alkylated with an alkylating agent, preferably methyl iodide or trimethyl or triethyl oxonium tetrafluoroborate, and then with guanidine in an alcoholic solvent, preferably methanol, ethanol or isopropanol, to obtain the compound of the Formula XI.

The compound of the Formula XI is hydrolyzed under basic conditions to form a compound of the Formula XII: 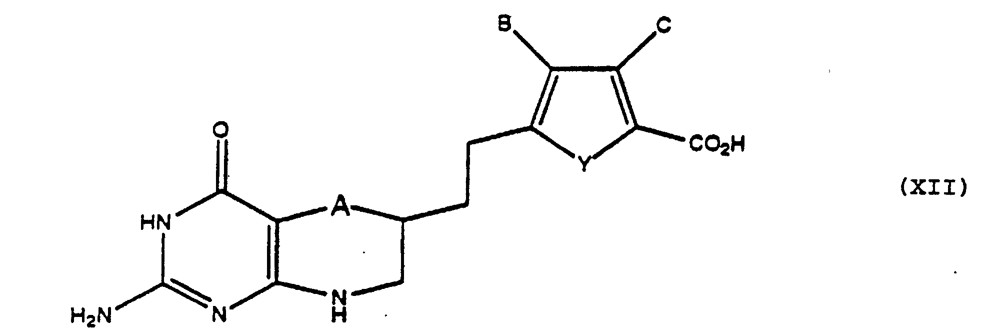 wherein A, B, C and Y are as defined above for the compoud of the present invention Where R₆ is hydrogen in the compound of the Formula XI, then the hydrolyzation reaction is not necessary, and the compound of the Formula XI is peptide coupled as described below.

The compound of the Formula XII (or the compound of the Formula XI where R₆ is hydrogen), which is in the free carboxylic acid form, can be peptide coupled, by means well known to those skilled in the art, with a glutamic acid diester hydrochloride to form a diester of the Formula XIII: 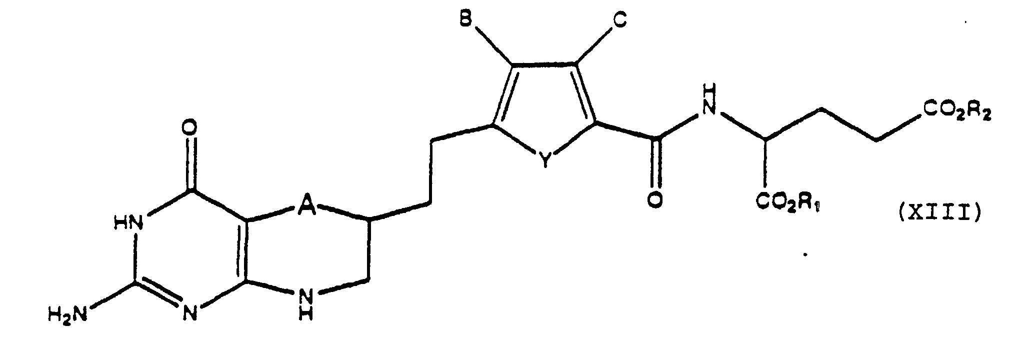 wherein A, B, C and Y are as defined above for the compound of the present invention and R₁ and R₂ are each independently a moiety that forms with the attached CO₂ a readily hydrolyzable ester group, such as a C₁-C₆ alkyl, hydroxyalkyl, alkylaryl or arylalkyl.

Finally, the compound of the Formula XIII is hydrolyzed to the free glutamic acid form of the coumpound of the present invention (R₁ and R₂ are each H).

Compounds of the invention contain one or more chiral centers. The invention encompasses racemic mixtures and mixtures of diastereomers, as well as optically active compounds, such as compounds essentially free of other optical isomers, which optically active compounds can be obtained by means known to those skilled in the art.

A detailed synthesis of a preferred compound of the present invention is presented in the example below.

### Example: 4-[2-(2-Amino-4-oxo-4,6,7,8-tetrahydro-3H-pyrimido[5,4-6][1,4]thiazin-6-yl)-ethyl]-2,5-thienoylamino-L-glutamic acid (Compound 1)

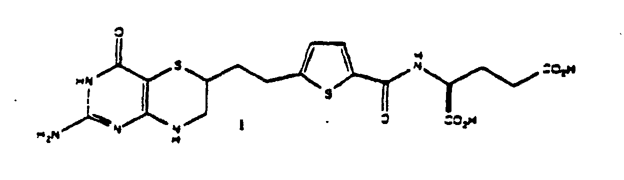

### Preparation of Methyl-5-bromo-2-thiophenecarboxlate 2:

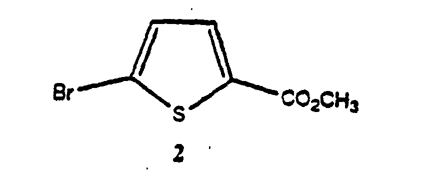

Compound **2** (CAS Reg. No. [62224-19-5]) was prepared as described at S. Gronowitz, Ark. *Kemi* 8, 1955, 87, and S. O. Lawesson, *Ark. Kemi* 11, 1957, 337.

### Preparation of 5-Ethynyl-2-carbomethoxy thiophene 3:

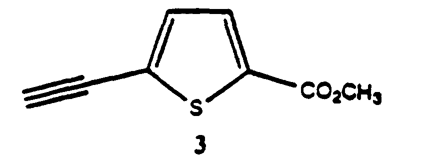

To a stirred solution of 5 g (22.7 mmol) of the bromide **3**, 160 mg (0.23 mmol) of tris-triphenylphosphine palladium(II) chloride and 65 mg (0.34 mmol) of cuprous iodide in 75 ml of diethylamine was added 4.8 ml (34 mmol) of trimethylsilyl acetylene. The resultant solution was stirred at 25°C. After 18 hours, the solvent was removed under reduced pressure, and the crude residue was dissolved in 300 ml Et₂O (diethyl ether) and extracted with 100 ml of IN HCl and 100 ml of saturated bicarbonate solution. The organic layer was dried (Na₂SO₄) and the solvent was removed under reduced pressure. The crude residue was flash chromatographed on silica gel with 5% Et₂O/hexanes to give 4.4 g (81% yield) of the silyl-protected product as a yellow solid. This material was used directly in the next step.

To a solution of 4.4 g (18.5 mmol) of the above silyl-protected acetylene in 50 ml of dimethylformamide (DMF) was added 3.3 g (35.2 mmol) of potassium fluoride monohydrate. After 15 minutes at 25°C, the mixture was poured into 500 ml of Et₂O and extracted four times with 100 ml of water (4 x 100 ml H₂O). The organic solution was dried (Na₂SO₄), and the solvent was removed under reduced pressure. The crude residue was flash chromatographed on silica gel with 10% Et₂O/hexanes to give 2.65 g (86% yield) of the desired product **3** as an orange solid. NMR (CDCl₃) δ: 3.45 (s, 1H), 3.88 (s, 3H), 7.22 (d, 1H, J=3.5Hz), 7.65 (d, 1H, J=3.5Hz). Anal. Calcd. for C₈H₆SO₂: C, 57.81; H, 3.64; S, 19.29. Found: C, 57.91; H, 3.71; S, 19.18.

An alternate method for the removal of the silyl group was also used as follows. To a stirred suspension of 3.7 g (0.02 mol) of anhydrous K₂CO₃ in 700 ml of methanol was added 59.7 g of the silyl-acetylene prepared above as a solid. The suspension was stirred for 2 hours at 35°C, and then the methanol was evaporated. The residue was added to 50 ml of water and this was extracted with 3 x 150 ml Et₂O, and the combined organic fractions were dried (Na₂SO₄) and evaporated. The crude residue was chromatographed on flash silica gel using 7% Et₂O/hexanes to give the desired product 3 in 98% yield (38.72 g).

### Preparation of N-(t-Butoxycarbonyl-glycinal 4:

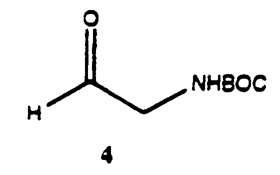

To a stirred solution of 2 g (12.4 mmol) of N-(t-butoxycarbonyl)-2-hydroxy-ethylamine (obtained from Sigma Chemicals) in 80 ml of CH₂Cl₂ at -78°C was added 1.3 ml (18.6 mmol) of dimethyl sulfoxide (DMSO) and 1.2 ml (13.7 mmol) of oxalyl chloride. After 5 minutes, 5.4 ml (38.4 mmol) of triethylamine was added and the solution was allowed to warm to 25°C. The mixture was poured into 200 ml of Et₂O and extracted with 100 ml of water, 100 ml of 0.5N HCl, and 100 ml of saturated bicarbonate solution. The organic fraction was dried (Na₂SO₄), and the solvent was removed under reduced pressure. The crude aldehyde was dissolved in 100 ml benzene, and the solvent was again removed under reduced pressure. This crude material, which was found to be unstable, was used immediately in the next step.

### Preparation of 4-N-(t-butoxycarbonyl)-3-hydroxy-1-(2-carbomethoxy-5-thiophene)-butyne 5:

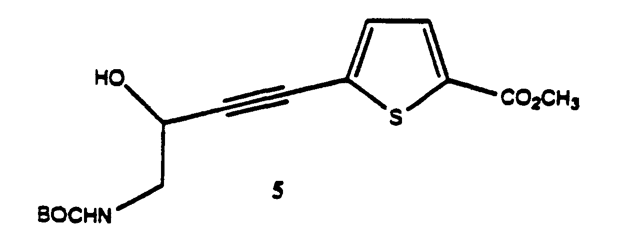

To a solution of 1.7 g (10.2 mmol) of acetylene **3** in 10 ml of dry tetrahydrofuran (THF) at -78°C was added 14.3 ml (14.0 mmol) of a 1M solution of lithium bis-trimethylsilyl amide in THF. After 10 minutes, the crude aldehyde **4** from above was added dropwise in 5 ml of dry THF. The solution was stirred at -78°C for 10 minutes and allowed to warm to 0°C over a twenty-minute period. To this was added 5 ml of a saturated aqueous solution of ammonium chloride. The mixture was poured into 200 ml of Et₂O and washed with 100 ml of H₂O and then 50 ml of saturated NaCl solution. The organic layer was dried (Na₂SO₄) and the solvent was removed under reduced pressure. The crude residue was flash chromatographed on silica gel with 10-40% EtOAc/hexanes to give 0.96 g (29%) of the desired alcohol **5** as a slightly yellow oil. NMR (CDCl₃) b: 1.46 (s, 9H), 3.38 and 3.58 (AB, 2H, J=3, 7Hz), 3.88 (s, 3H), 4.71 (dt, 1H, J=2.9, 5.2Hz), 5.05 (brs, 1H), 7.15 (d, 1H, J=3.9Hz), 7.64 (d, 1H, J=3.9Hz). IR (neat): 2978.3, 1729.5, 1685, 1523, 1452, 1368, 1286, 1167, 1098, 959, 822, 750.4 cm⁻¹ . High Resolution Mass Spec., Calcd. for C₁₅H₁₉NO₅S : M⁺Cs⁺, 458.0038. Found: 458.0051.

Preparation of Methyl-5-[(4-N-(t-butoxycarbonyl)-amino-3-hydroxy)-butyl]-thienyl-2-carboxylate **6**:

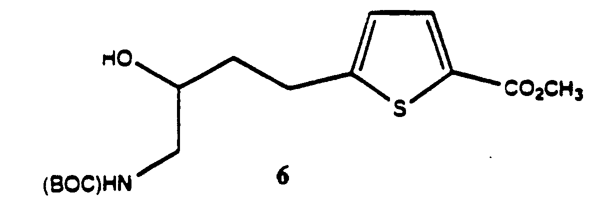

To a stirred solution of 940 mg (2.89 mmol) of the above acetylene **5** in 50 ml of ethylacetate (EtOAc) was added 320 mg of 5% Pd/C. The mixture was placed under 40 psi of H₂ gas and stirred at 25°C for 17 hours. The mixture was filtered through Celite (diatomaceous earth) and the solvent was removed under reduced pressure. The crude residue was flash chromatographed on silica gel with 20% EtOAc/CH₂Cl₂ to give 800 mg (84% yield) of the desired alcohol as a yellow oil. NMR (CDCl₃) 6: 1.44 (s, 9H), 1.81 (m, 2H), 2.28 (brs, 1H), 2.99 (m, 2H), 3.10 and 3.29 (AB, 2H, J=3, 6.9Hz), 3.74 (m, 1H), 3.86 (s, 3H), 4.89 (brs, 1H), 6.82 (d, 1H, J=3.7Hz), 7.63 (d, 1H, J=3.7Hz). Anal. Calcd. for C₁₅H₂₃NO₅S: C, 54.69; H, 7.04; N, 4.25; S, 9.73. Found: C, 54.79; H, 7.02; N, 4.29; S, 9.63.

An alternate method for the preparation of alcohol **6** uses bis-t-butoxycarbonyl allylamine **14** as follows.

### Preparation of Bis-t-butoxycarbonyl allylamine 14:

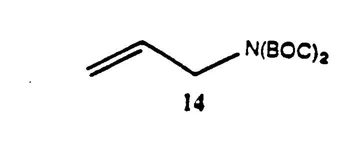

To a solution of 57.10 g (1.0 mol) of allylamine and 1.2 g (0.01 mol) of dimethylaminopyridine (DMAP) in 500 ml of acetonitrile was added a solution of 220 g (1 mol) of (t-BOC)₂O in 100 ml of acetonitrile, and the resultant mixture was stirred for 6 hours. The reaction mixture was diluted with toluene (100 ml) and the solvents were evaporated under reduced pressure at 60°C. The resultant oil was re-dissolved in acetonitrile (400 ml) and another 1.2 g (0.01 mol) of DMAP was added, and to this a solution of 220 g (1 mol) of (t-BOC)₂O in 100 ml of acetonitrile was slowly added. The reaction mixture was stirred for 12 hours at 60°C, the solvent was evaporated under reduced pressure at 60°C, and dilute NaHCO₃ (100 ml) was added. This was extracted with 3 x 150 ml CH₂Cl₂, and the combined organic layers were washed with brine, dried (Na₂SO₄) and evaporated. The crude residue was purified by chromatography on flash silica gel eluting with a gradient of 5-20% EtOAc/hexanes to give 156.9 g (63% yield) of the desired product **14** as a clear crystalline solid (m.p. 43-44°C). ¹H NMR (CDCl₃) δ (ppm) : 1.5 (s, 18H), 4.18 (dd, 2H, J=15 Hz, J=1 Hz), 5.14 (ddd, 2H, J=15 Hz, J=10 Hz, J=1 Hz), 5.85 (ddt, 2H, J=10 Hz, J=5 Hz, J=1 Hz). IR (KBr): 2978, 2935, 2860, 1724, 1689, 1342, 1130 cm⁻¹. Anal. Calcd. for C₁₃H₂₃NO₄: C, 60.68; H, 9.01; N, 5.44. Found: C, 60.78; H, 9.04; N, 5.50.

### Preparation of 1-(bis-t-butoxycarbonyl amino)-2-ethanal 15:

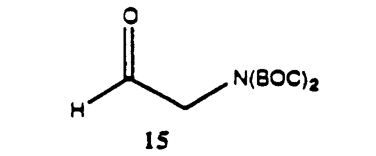

A solution of 0.60 g (2.34 mmol) of bis-t-butoxycarbonyl allylamine **14** in 20 ml of CH₂Cl₂ was ozonized (40 volts, 500 amperes, 1.0 1/min. O₂ @ 3 psi) at -78°C until a blue color persisted, 10 ml of dimethyl sulfide was added, and this mixture was stirred for 14 hours at 25°C. The mixture was added to dilute brine and extracted with CH₂Cl₂ (3 x 50 ml), and the organic fractions were dried (Na₂SO₄) and evaporated. Chromatography using flash silica gel yielded 603 mg (99% yield) of the desired product **15** as a clear crystalline solid (m.p. 37-39°C). ¹H NMR (CDCl₃) δ (ppm): 1.50 (s, 18H), 4.38 (s, 2H), 9.55 (s, 1H). IR (KBr) : 2984, 2935, 2724, 1792, 1734, 1699, 1362, 1153 cm⁻¹. Anal. Calcd. for C₁₂H₂₁NO₅ : C, 55.58; H, 8.16; N, 5.40. Found: C, 55.20; H, 8.19; N, 5.19.

### Preparation of Methyl-5-[(4-N-(t-butoxycarbonyl)-amino-3-hydroxy)-butyl]-thienyl-2-carboxylate 6:

To a solution of 9.64 g (59 mmol) of 5-ethynyl-2-carbomethoxy thiophene **3** in THF (250 ml) at -78°C was added 65.6 ml (60 mmol, 0.9M) of lithium hexamethyl disilazide (LiHMDS), and the mixture was stirred for 2 hours at -78°C. A solution of 15.6 g (60 mmol) of 1-(bis-t-butoxyxcarbonylamino)-2-ethanal **15** in THF (40 ml) was cannulated into the reaction mixture, and the mixture was stirred for 8 hours at -78°C. A solution of 3.4 ml (60 mmol) acetic acid in methanol (10 ml) was added as a quench, the mixture was stirred for 10 minutes warming to 0°C, and water (60 ml) was added. This was extracted with EtOAc (3 x 100 ml), and the organic extracts were washed with dilute NaHCO₃ and dried (Na₂SO₄). The solvent was removed by reduced pressure to approximately 50 ml. To the resulting residue was added EtOAc (125 ml), and this solution was added to a Parr bottle containing 7.38 g (30 wt. % of acetylene starting material) of 5% Pd on carbon and hydrogenated at 55 psi of H₂ for 24 hours. The crude hydrogenation material was filtered through Celite, and the filter cake was washed with methanol (100 ml) and EtOAc (100 ml). The solvent was evaporated under reduced pressure, the crude residue was filtered through silica eluting with 50% EtOAc in hexanes, and the solvent was evaporated. The residue was solvated in 20 ml of methanol (MeOH) azeotroped with benzene (50 ml), and re-dissolved in dry MeOH (20 ml). This mixture was added to a freshly prepared solution of 60 ml (2M) of sodium methoxide in methanol. The reaction mixture was stirred for 45 minutes, dilute HCl (0.1M, 50 ml) was added, and this mixture was extracted with EtOAc (3 x 75 ml). The organic fractions were combined and washed with pH 7 buffer (1M), dried (Na₂SO₄) and evaporated. The crude residue was chromatographed on flash silica eluting with 40% EtOAc/hexanes to give 9.32 g (48% yield) of the desired product 6.

### Preparation of Methyl-5-[(4-N-(t-butoxycarbonyl)-amino-3-acetylthio) -butyl]-2-thiophenecarboxylate 7:

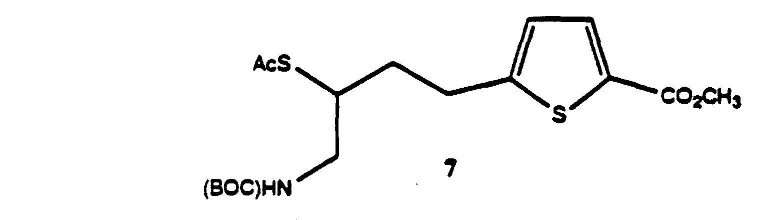

To a stirred solution of 9.26 g (28.1 mmol) of the alcohol **6** in 100 ml of THF at 0°C was added 5.9 ml (42 mmol) of triethylamine (TEA) and 2.4 ml (31 mmol) of methanesulfonyl chloride. After 20 minutes, 100 ml of DMF and 12.8 g (110 mmol) of potassium thioacetate were added, and the resultant solution was allowed to warm to 25°C. After three days, the mixture was poured into 500 ml of water and extracted with 800 ml of Et₂O. The organic layer was washed with 200 ml of water, 200 ml of 1N HCl, 200 ml of saturated bicarbonate solution, and 100 ml of saturated NaCl solution. The organic layer was dried (MgSO₄) and the solvent was removed under reduced pressure. The crude residue was flash chromatographed on silica gel with 25% EtOAc/hexanes to give 10.3 g (95% yield) of the desired thioacetate **7** as a yellow oil. NMR (CDCl₃) δ: 1.44 (s, 9H), 1.91 and 2.04 (ABm, 2H), 2.37 (s, 3H), 2.96 (m, 2H), 3.36 (m, 2H), 3.61 (m, 1H), 3.86 (s, 3H), 4.74 (brs, 1H), 6.79 (d, 1H, J=3.6Hz), 7.62 (d, 1H, J=3.6Hz). IR (neat): 3366, 2976.4, 1713, 1520, 1462.1, 1366, 1290.5, 1267.3, 1169, 1098, 752, 631 cm⁻¹. High Resolution Mass Spec., Calcd. for C₁₇H₂₅NO₅S₂: M⁺Cs⁺, 520.0229. Found: 520.0240.

Preparation of Methyl-5-[(4-N-(t-butoxycarbonyl)-amino-3-(dimethylmalonyl)thio)-butyl]-2-thiophenecarboxylate **8**:

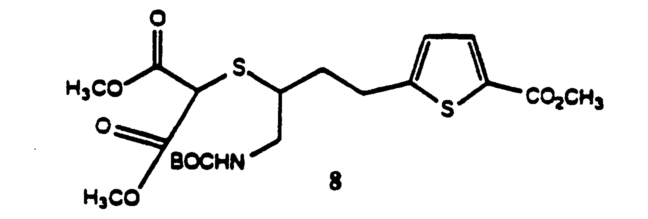

To a stirred solution of 10.2 g (26 mmol) of the above thioacetate **7** in 200 ml of dry methanol at 0°C was added 7.2 g (52 mmol) of K₂CO₃ and 3.7 ml (29 mmol) of dimethyl chloromalonate. After three hours, the mixture was poured into 500 ml of water and extracted with Et₂O (3 x 500 ml). The combined organic layers were washed with 500 ml of water and then with 200 ml of saturated NaCl solution, and dried (MgSO₄) . The solvent was removed under reduced pressure and the crude residue was flash chromatographed on silica gel with 30% EtOAc/hexanes to give 11.46 g (93% yield) of the desired thioether **8** as a slightly yellow oil. NMR (CDCl₃) δ: 1.44 (s, 9H), 1.85 and 2.00 (ABm, 2H), 3.03 (m, 3H), 3.30 (m, 2H), 3.80 (s, 6H), 3.86 (s, 3H), 5.10 (brs, 1H), 6.82 (d, 1H, J=3.8Hz), 7.63 (d, 1H, J=3.8Hz). IR (neat): 3442, 2974, 2955, 1734, 1713, 1512, 1460, 1435, 1366, 1291, 1267, 1167, 1098, 1022, 752 cm⁻¹. High Resolution Mass Spec., Calcd. for C₂₀H₂₉NO₈S₂: M⁺Cs⁺, 608.0389. Found: 608.0370.

### Preparation of 6-[(5-Carbomethoxythien-2-yl)-ethyl]-2-carbomethoxy-3-oxo-3,4,5,6-tetrahydro-[1,4]-thiazine 9:

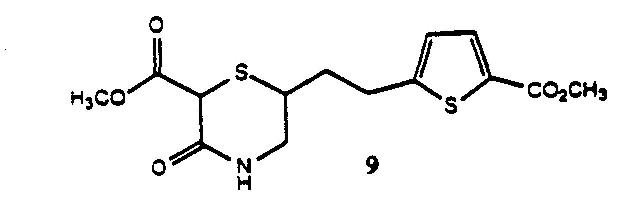

To a stirred solution of 470 mg (0.99 mmol) of the above thioether **8** in 12 ml of CH₂Cl₂ at 0°C was added 4 ml or trifluoroacetic acid (TFA). After 1.5 hours, the mixture was poured into 50 ml of saturated bicarbonate solution and extracted with CH₂Cl₂ (4 x 100ml). The solvent was removed under reduced pressure and the crude residue was dissolved in 10 ml of methanol. After stirring at 25°C for 1.5 hours, the solvent was removed under reduced pressure and the crude residue was flash chromatographed on silica gel with 40% EtOAc/CH₂Cl₂ to give 298 mg (88% yield) of the desired lactam **9** as a colorless oil. NMR (CDCl₃) δ: 1.94 (m, 2H), 3.01 (m, 2H), 3.4-3.7 (m, 4H), 3.80 and 3.82 (s, 3H), 3.87 (s, 3H), 6.25 (brs, 1H,), 6.83 (d, 1H, J=3.7Hz), 7.64 (d, 1H, J=3.7Hz). IR (KBr): 2951, 1732, 1669, 1462, 1294, 1267, 1194, 1157, 1098, 1005, 752 cm⁻¹. Anal. Calcd. for C₁₄H₁₇NO₅S₂: C, 48.96; H, 4.99; N, 4.08; S, 18.67. Found: C, 49.06; H, 4.93; N, 4.09; S, 18.60.

### Preparation of Methyl-[2-(2-amino-4-oxo-4,6,7,8-tetrahydro-3H-pyrimido[5,4-6][1,4]thiazin-6-yl)-ethyl]-2,5-thienoate 10:

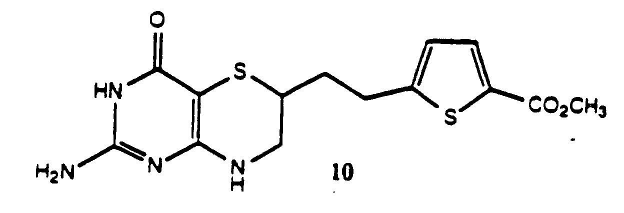

To a stirred solution of 1.9 g (5.5 mmol) of the above lactam **9** in 150 ml of dry CH₂Cl₂ was added, all at once, 1.06 g (7.2 mmol) of trimethyloxonium tetrafluoroborate. The solution was stirred for 6 hours at 25°C. The resulting mixture was poured into 50 ml of 10% aqueous K₂CO₃ and extracted with CH₂Cl₂ (3 x 200 ml). The combined organic layers were dried (MgSO₄) and the solvent was removed under reduced pressure. The resulting material was used directly in the next step.

In a separate flask was placed 1.58 g (16.6 mmol) of dry guanidine hydrochloride. To this was added 600 ml of dry methanol. Dry argon was bubbled through the solution for 10 minutes, after which was added 926 mg (17.1 mmol) of dry sodium methoxide. To the resultant mixture was added via a cannula the crude lactim ether prepared above in 20 ml MeOH. The solution was refluxed under an argon atmosphere for 20 hours. The pH was adjusted to 4 with 1N HCl, and the solvent was removed under reduced pressure. The crude residue was dissolved in 500 ml of CHCl₃ and washed with 2 x 100 ml of water. The organic layer was dried (MgSO₄), and the solvent was removed under reduced pressure. The crude residue was flash chromatographed on silica gel with 5-10% MeOH/CH₂Cl₂ to give 413 mg of an off-white solid. This material was then dissolved in 25 ml of hot MeOH and slowly cooled to 4°C over an 18-hour period. The solid was collected by filtration to give 180 mg (9% yield) of the desired pyrimidinone **10** as a slightly yellow solid. NMR (d₆-DMSO) 5: 1.72 (m, 1H), 1.88 (m, 1H) , 2.8-3.22 (m, 4H), 3.50 (dt, 1H. J=2, 8Hz), 5.99 (brs, 2H), 6.64 (brs, 1H). 6.98 (d, 1H, J=3.8Hz), 7.62 (d, 1H, J=3.8Hz), 10.02 (brs, 1H). High Resolution Mass Spec., Calcd. for C₁₄H₁₆N₄O₃S₂: M⁺Na⁺, 375.0562. Found: 375.0550.

An alternate procedure for the preparation of pyrimidinone **10** was also used as follows. To a stirred solution of 500 mg (1.46 mmol) of the lactam **9** in 20 ml of dry THF was added 648 mg (1.60 mmol) of Lawesson's reagent (2,4-bis(4-methoxyphenyl)-1,3-dithia-2,4-diphosphetane-2,4-disulfide). After the mixture was stirred at 25°C for 20 hours, it was poured into 50 ml of saturated bicarbonate solution and extracted with 2 x 200 ml of EtOAc. The combined organic layers were washed with 50 ml of saturated NaCl solution and dried (MgSO₄), and the solvent was removed under reduced pressure. The crude thiolactam was flash chromatographed on silica gel with 8% EtOAc/CH₂Cl₂ to give 525 mg of the corresponding thiolactam **11**: 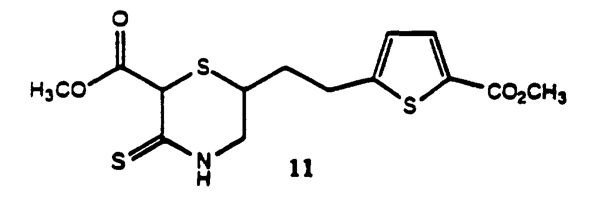 This material was used immediately in the next step.

To the 525 mg of the thiolactam **11** in a mixture of 10 ml of dry THF and 10 ml of dry methanol was added 1.6 ml of 1N NaOH. To this was added 0.1 ml (1.6 mmol) of methyl iodide to produce a methylated thiolactam, which was used as described below without purification. In a separate flask was placed 1.39 g (14.6 mmol) of dry guanidine hydrochloride. To this was added 300 ml of dry methanol. Dry argon was bubbled through the solution for 10 minutes, after which was added 796 mg (14.7 mmol) of dry sodium methoxide; To the resultant mixture was added via a cannula the crude methylated thiolactam prepared above. The solution was refluxed under an argon atmosphere for 48 hours. The pH was adjusted to 4 with 1N HCl and the solvent was removed under reduced pressure. The crude residue was dissolved in 500 ml of CHCl₃ and washed with 2 x 100 ml of water. The organic layer was dried (MgSO₄) and the solvent was removed under reduced pressure. The crude residue was flash chromatographed on silica gel with 5-10% MeOH/CH₂Cl₂. The material was then dissolved in 20 ml of hot MeOH and slowly cooled to 4°C over an 18-hour period. The solid was collected by filtration to give 131 mg (9% yield) of the desired pyrimidinone **10**.

### Preparation of [2-(2-Amino-4-oxo-4,6,7,8-tetrahydro-3H-pyrimido[5,4-6][1,4] thiazin-6-yl)-ethyl]-2,5-thienoic acid 12:

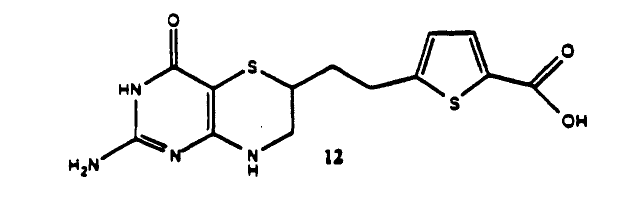

To 180 mg (0.51 mmol) of the pyrimidinone **10** was added 5 ml of 1N NaOH in water. The resulting solution was stirred at 25°C for 20 hours. After cooling to 0°C, the pH was adjusted to 2 with 1N HCl. The brown solid was filtered off and washed with water and dried under vacuum to give 111 mg (64% yield) of the desired acid **12** as a slightly brown solid. NMR (d₆-DMSO) δ: 1.72 (m, 1H), 1.92 (m, 1H), 2.78-3.68 (m), 6.07 (brs, 2H), 6.68 (brs, 1H), 6.92 (d, 1H, J=3.7Hz), 7.52 (d, 1H, J=3.7Hz), 10.12 (brs, 1H), 12.89 (brs, 1H). High Resolution Mass Spec., Calcd. for C₁₃H₁₄N₄O₃S₂: M⁺, 338.0507. Found: 338.0517.

### Preparation of 4-[2-(2-Amino-4-oxo-4,6,7,8-tetrahydro-3H-pyrimido[5,4-6] [1,4] thiazin-6-yl)-ethyl] -2,5-thienoylamino-L-glutamic acid diethyl ester 13:

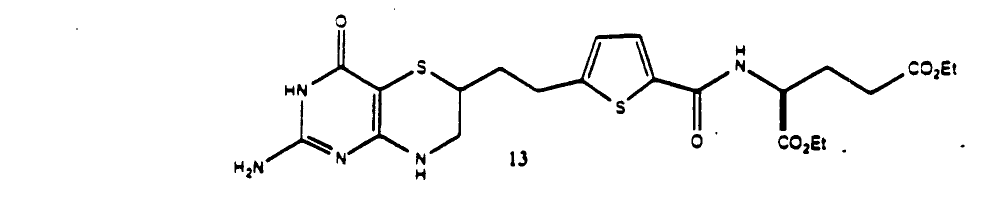

To a stirred solution of 110 mg (0.33 mmol) of the above acid **12** in 5 ml of dry DMF was added 48 mg (0.36 mmol) of 1-hydroxy-benzotriazole hydrate, 62 µl (0.36 mmol) of diisopropylethylamine, 86 mg (0.36 mmol) of glutamic acid diethyl ester hydrochloride, and 106 mg (0.36 mmol) of 1-(3-dimethylaminopropyl)-3-ethylcarbodiimide methiodide. The resultant solution was stirred under an argon atmosphere at 25°C for 20 hours and then poured into 50 ml of water. The water was extracted with 2 x 150 ml of EtOAC. The combined organic layers were back-extracted with 3 x 50 ml of water and then 10 ml of saturated NaCl solution, and dried (MgSO₄). The solvent was removed under reduced pressure, and the crude residue was flash chromatographed on silica gel with 0-10% MeOH/CH₂Cl₂ to give 120 mg (71% yield) of the desired amide **13** as a slightly yellow amorphous solid. NMR (d₆-Acetone) δ: 1.20 (m, 6H), 2.2 (m, 1H), 2.46 (t, 1H, J=7.6Hz), 2.78 (s, 1H), 2.82 (s, 1H), 2.92-3.1 (m, 2H), 3.41 (m, 1H), 3.72 (dt, 1H, J=3.0, 12.7Hz), 4.02-4.2 (m, 4H), 4.58 (m, 1H), 5.90 (brs, 1H), 6.05 (brs, 1H), 6.90 (d, 1H, J=3.8Hz), 7.58 (d, 1H, J=3.8Hz), 7.78 (d, 1H, J=8Hz). High Resolution Mass Spec., Calcd. for C₂₂H₂₉N₅O₆S₂: M⁺H⁺, 524.1638. Found: 524.1650.

### Preparation of 4-[2-(2-Amino-4-oxo-4,6,7,8-tetrahydro-3H-pyrimido(5,4-6) [1,4)thiazin-6-yl)-ethyl] -2,5-thienoylamino-L-glutamic acid 1:

To 115 mg (0.22 mmol) of the above diethyl ester 13 was added 3 ml of 1N NaOH solution in water. The resulting mixture was stirred at 25°C for 14 hours. After cooling to 0°C, the pH was adjusted to 3.5 with aqueous HCl. The solid was filtered, washed with water and dried under vacuum to give 82 mg (80% yield) of the desired acid as an off-white solid. NMR (d₆-DMSO) δ: 1.62-2.02 (m, 4H), 2.27 (m, 2H), 2.78-3.00 (m, 3H), 4.27 (dd, 1H, J=6, 6.8Hz), 6.00 (brs, 2H), 6.63 (brs, 1H), 6.88 (d, 1H. J=3.7Hz), 7.63 (d, 1H, J=3.7Hz), 8.37 (d, 1H, J=7.4Hz), 10.05 (brs, 1H), 12.85 (brs, 2H). IR (KBr): 3371, 1700, 1643, 1543, 1345 cm⁻¹. High Resolution Mass Spec., Calcd. for C₁₈H₂₁N₅O₆S₂: M⁺H⁺, 468.1012. Found: 468.1025. Anal. Calcd. for C₁₈H₂₁N₅O₆S₂.1.5 H₂O: C, 43.71; H, 4.89; N, 14.16; S, 12.97. Found: C, 43.50; H, 4.67; N, 14.07; S, 12.72.

### Biological and Biochemical Evaluation

### Determination of Inhibition Constants for GAR Transformylase:

The GAR-transformylase (GARFT) assay method of Young et al., *Biochemistry* 23 (1984), 3979-3986, was modified and used as described below. Reactions mixtures contained the catalytic domain of the human GARFT, 0-250 nM of Compound 1, 20 µM glycinamide ribonucleotide (GAR), 10 or 20 µM N¹⁰-formyl-5,8-dideazafolate (FDDF), 50 mM HEPES-KOH, pH 7.5, and 50 mM KCl. The reaction was initiated with the addition of enzyme to a final concentration of 11 nM, followed by monitoring of the increase in absorbance at 294 nm at 20°C (e₂₉₄ = 18.9 mM⁻¹ cm⁻¹).

The GARFT inhibition constant (Kᵢ) was determined from the dependence of the steady-state catalytic rate on inhibitor and substrate concentration. The type of inhibition observed was determined to be competitive with respect to FDDF by the dependence of the apparent Kᵢ (Kᵢ, app) on the concentration of FDDF and was shown to be described by Kᵢ, app = Kᵢ + (Kᵢ/Kₘ) [FDDF]. The Michaelis constant for FDDF, Kₘ, was determined independently by the dependence of the catalytic rate on FDDF concentration. Data for both the Kₘ and Kᵢ determinations were fitted by nonlinear methods to the Michaelis equation, or the Michaelis equation for competitive inhibition, as appropriate. Data resulting from tight-binding inhibition was analyzed, and Kᵢ determined, by fitting the data to the tight-binding equation of Morrison, *Biochem Biophys Acta* 185 (1969), 269-286, by nonlinear methods.

### Cell lines:

The cell lines used and their origin are tabulated in Table 1. The growth conditions and media requirements of each cell line are summarized in Table 2. All cultures were maintained at 37°C, 5% air-CO₂ in a humidified incubator.

### In vitro growth inhibition:

Stock solutions of the inhibitors were prepared in 10 mM sodium bicarbonate in water and stored in 1 ml aliquots at - 20°C for cell culture experiments. Cell growth inhibition was measured by a modification of the method of Mosmann, *J*. *Immunol.* Methods 65 (1983), 55-63.

Mid-log phase cells of each cell line were diluted to 18,500 cells/ml in fresh RPMI growth medium (Mediatech, Washington, DC) supplemented with dialyzed fetal calf serum (Hyclone Laboratories Inc., Logan, UT) and then aliquotted into columns 2 through 12 of 96-well microtiter plates. Column 1 was filled with the same volume (135 ml) of fresh medium, without cells, for use as a blank. The plates were then placed in a 37°C, 5% air-CO₂ incubator. After 1 to 4 hours, plates were removed from the incubator followed by addition of Compound **1** at 10 x final concentration, 15 ml/well in binary dilutions, to columns 12 to 4. For reversal experiments, hypoxanthine (1.75 mM) or AICA (1.75 mM) was included in all drug solutions (final concentration 175 mM). Wells containing each concentration of Compound **1** were prepared in quadruplicate on each plate. Fifteen milliliters of media, without Compound **1**, were added to the wells in column 1 of the plates. The cells were then returned to the incubator and remained undisturbed for the full incubation period. On day 3 for L1210 and L1210/CI920 cells, or day 5 for CCRF-CEM cells, 50 ml of 0.8 mg/ml MTT ((4,5-dimethylthiazol-2-yl)-2,5-diphenyl tetrazolium bromide; Sigma catalog no. M2128) dissolved in tissue culture medium was added to each well of all plates, after which the cells were returned to the incubator. After 4 hours, all plates were removed from the incubator and centrifuged at 1200 rpm for 7 minutes. Media were siphoned off, and 150 ml of DMSO was added to each well of all plates. Plates were then mixed at slow speed on a vortex mixer for one hour in the dark at room temperature. The extent of metabolized MTT was measured spectrophotometrically at 540 nm on a Molecular Devices Vmax kinetic microplate reader. The concentration of drug required to reduce cell growth by 50% as measured by MTT metabolism was determined by interpolation between the O.D. (minus blank) immediately above and below 50% of control O.D. (minus blank).

**TABLE 1**

| Tissue of Origin and Source of Cell Lines Employed in *In Vitro* Studies | | |
|---|---|---|
| Cell Line | Source | Origin |
| L1210 | ATCC^{#} | Mouse, lymphocytic leukemia |
| CCRF-CEM | ATCC^{#} | Human, acute lymphoblastic leukemia |
| ^{#}ATCC = American Type Culture Collection | | |

**TABLE 2**

| Culture Conditions, Plating Densities and Incubation Times Used in Microtiter Assays | | | | |
|---|---|---|---|---|
| Cell line | Medium | DFCS Cone.* % | Plating Density (cells/well) | Incubation Time (days) |
| L1210 | RPMI-1640 | 5 | 2500 | 3 |
| CCRF-CEM | RPMI-1640 | 10 | 2500 | 5 |

| | | | | |
|---|---|---|---|---|
| *DFCS Conc. = dialyzed fetal calf serum concentration. | | | | |

**TABLE 3**

| Data for Compound 1 Growth Inhibition Using Continuous (72-hour) Exposure | | | |
|---|---|---|---|
| Compound | GARFT Kᵢ (nM) | IC₅₀ Cell Culture L1210 (nM)^{a} | IC₅₀ Cell CultureCCRF-CEM (nM)^{a} |
| 1 | 4.5 | 16 | 4.3 |

| | | | |
|---|---|---|---|
| ^{a}: Mean IC₅₀ ± standard deviation. | | | |

### In Vivo Antitumor Activity:

On day zero, 2 mm² trocar fragments of 6C3HED lymphosarcoma were implanted subcutaneously in the axillary region into groups of six C3H/He female mice. Starting on day 1, the test compound was administered intraperitoneally in 40% Encapsin given once daily for nine days. Control animals received identical treatment without test compound. The percentage inhibition given in Table 4 below was calculated by comparing the mass of the control tumors (receiving diluent only) on day 11 to those of animals receiving test compound.

**TABLE 4**

| Compound | Dose (mg/kg) | % Inhibition | Survivors |
|---|---|---|---|
| **1** | 12.5 | 100 | 6/6 |
| **1** | 25 | 100 | 3/6 |
| **1** | 50 | toxic | 0/6 |

On day zero, 2 mm² trocar fragments of C3H/BA mammary adenocarcinoma were implanted subcutaneously in the axillary region into groups of six C3H/He female mice. Starting on day 1, the test compound was administered intraperitoneally in 40% Encapsin given once daily for nine days. Control animals received identical treatment without test compound. The percentage of inhibition was calculated by comparing the mass of the control tumors (receiving diluent only) on day 14 to those of animals receiving test compound. The results are summarized in Table 5.

**TABLE 5**

| Compound | Dose (mg/kg) | % Inhibition | Survivors |
|---|---|---|---|
| **1** | 5 | 16 | 5/6 |
| **1** | 10 | 84 | 6/6 |
| **1** | 20 | 100 | 3/6 |

It will be apparent to those skilled in the art that various modifications and variations can be made in the processes and products of the present invention.

Where possible, as a matter of chemistry, chemical groups recited herein can be substituted. In some cases, this possibility is made explicit by reciting, e.g., substituted or unsubstituted C₁-C₃ alkyl group.

Where more than one R₆ group is recited in any Formula herein, each R₆ can be independently selected from the possibilities given.

## Claims

1. A compound of the formula 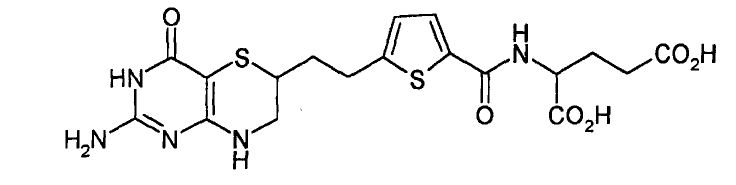 or a pharmaceutically acceptable salt of said compound.

2. A compound according to claim 1 which is optically active.

3. A compound according to claim 2 having the following structure.

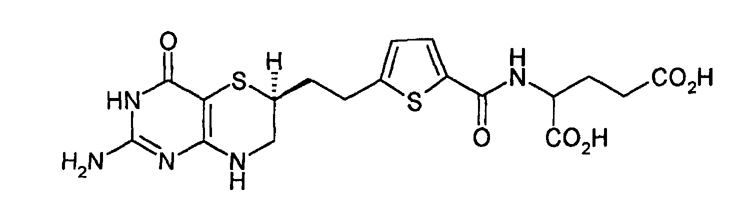

4. A compound according to claim 2 having the following structure.

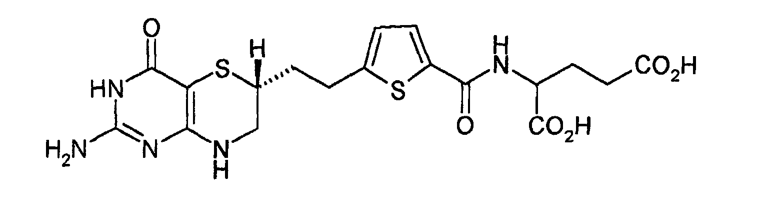

5. A pharmaceutical composition comprising:
(I) a compound according to any of the claims 1 to 4 or a pharmaceutically acceptable salt thereof;
(II) a pharmaceutically acceptable carrier, excipient or diluent.

## Patentansprüche

1. Eine Verbindung der Formel 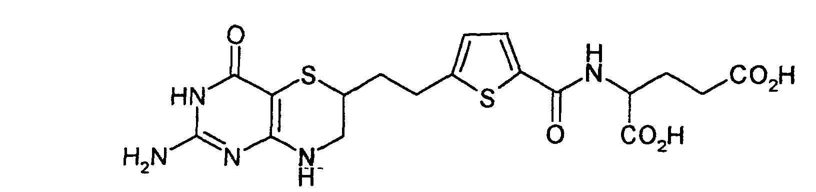 oder ein pharmazeutisch geeignetes Salz dieser Verbindung.

2. Eine Verbindung gemäß Anspruch 1, die optisch aktiv ist.

3. Eine Verbindung gemäß Anspruch 2, die die folgende Struktur besitzt.

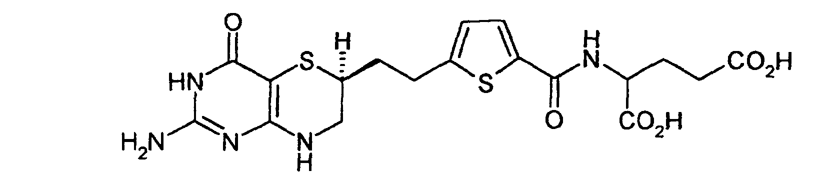

4. Eine Verbindung gemäß Anspruch 2, die die folgende Struktur besitzt.

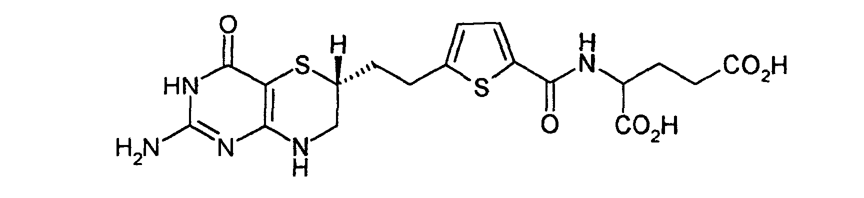

5. Eine pharmazeutische Zusammerietzung umfassend:
(I) eine Verbindung gemäß einem der Ansprüche 1 bis 4 oder ein pharmazeutisch geeignetes Salz davon;
(II) ein pharmazeutisch geeigneter Träger, Arzrieihilfsträger oder Verdünnungsmittel.

## Revendications

1. Composé de formule : 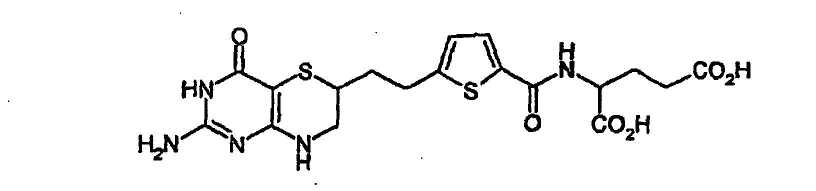 ou un sel acceptable sur le plan pharmaceutique dudit composé.

2. Composé selon la revendication 1, qui est optiquement actif.

3. Composé selon la revendication 2, ayant la structure suivante :

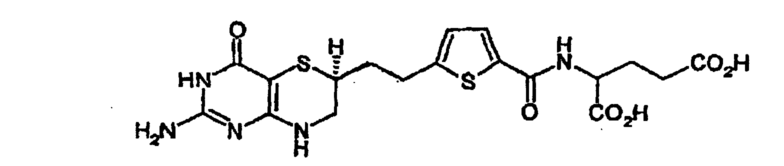

4. Composé selon la revendication 2, ayant la structure suivante :

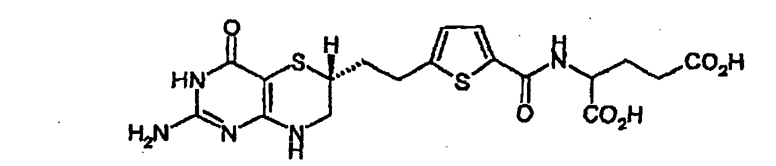

5. Composition pharmaceutique comprenant :
(I) un composé selon l'une quelconque des revendications 1 à 4 ou un sel acceptable sur le plan pharmaceutique de celui-ci ;
(II) un véhicule, un excipient ou un diluant acceptable sur le plan pharmaceutique.
